# FASCICULE DE BREVET EUROPEEN

(11) **EP 3 134 382 B1**
(45) Date de publication et mention de la délivrance du brevet: **08.01.2020**
(21) Numéro de dépôt: 15725794.0
(22) Date de dépôt: 24.04.2015
(51) Int. Cl.: C07C 69/92, A61K 8/37, A61Q 15/00

(54) **NOUVEAUX DÉRIVÉS DE BENZOATE DE BENZYLE**
NEUE DERIVATE VON BENZYLBENZOAT
NOVEL DERIVATIVES OF BENZYL BENZOATE

(30) Priorité: 25.04.2014 FR 1453738
(43) Date de publication de la demande: 01.03.2017
(73) Titulaire: Robertet S.A., 06130 Grasse (FR)
(72) Inventeur: PEGARD, Anthony, 06130 Grasse (FR); CASAZZA, André, 06130 Grasse (FR); KERVERDO, Raymond, 06460 St Vallier de Thiey (FR)
(74) Mandataire: Agasse, Stéphane
(86) Numéro de dépôt international: PCT/FR2015/051115
(87) Numéro de publication internationale: WO 2015/162392

(56) Documents cités:
- EP-A1- 0 169 246
- WO-A1-91/07165
- Chester J Cavallito ET AL: "Synthesis of Phenolic Acid Esters. I. Depsides", Journal of the American Chemical Society (1943), 65, 1 janvier 1943 (1943-01-01), pages 2140-2142, XP055154564, Extrait de l'Internet: URL:http://pubs.acs.org/doi/pdf/10.1021/ja 01251a034 [extrait le 2014-11-24]
- GUEN LE A ET AL: "SYNTHESIS AND PROPERTIES OF FLEXIBLE POLY(ETHER KETONE) BACKBONES, GRAFTED WITH STIFF, MONODISPERSE SIDE CHAINS", MACROMOLECULES, AMERICAN CHEMICAL SOCIETY, US, vol. 31, no. 19, 22 septembre 1998 (1998-09-22), pages 6559-6565, XP000778175, ISSN: 0024-9297, DOI: 10.1021/MA971640Y
- HAO LIN ET AL: "Synthesis and antibacterial evaluation of anziaic acid and its analogues as topoisomerase I inhibitors", MEDCHEMCOMM, vol. 4, no. 12, 1 janvier 2013 (2013-01-01), page 1613, XP055154453, ISSN: 2040-2503, DOI: 10.1039/c3md00238a

## Description

La présente invention a pour objet de nouveaux dérivés de benzoate de benzyle et l'utilisation de ces dérivés pour des applications cosmétiques, notamment comme déodorant.

La transpiration est un phénomène naturel et nécessaire qui contribue à réguler la température du corps.

Lors des phases de transpiration, les glandes sudoripares sécrètent la sueur qui contient essentiellement de l'eau, mais également des minéraux et du lactate.

Chez l'homme, on distingue deux sortes de glandes sudoripares qui diffèrent par leurs fonctions et par la composition de la sueur qu'elles excrètent :
- les glandes sudoripares « eccrines » ; et
- les glandes sudoripares « apocrines ».

Les glandes sudoripares eccrines sont de loin les plus nombreuses et se localisent sur presque tout le corps mais surtout sur la paume des mains, sur la plante des pieds et sur le front. Chacune d'elle est une glande simple, tubuleuse et en spirale, dont l'extrémité, le glomérule, se situe dans l'épaisseur du derme ou dans le tissu sous-cutané.

La sueur sécrétée par les glandes sudoripares eccrines est composée à 99 % d'eau et d'électrolytes, notamment du chlorure de sodium, et d'environ 1% de composés organiques, notamment d'acide lactique. Bien que la sueur sécrétée par les glandes sudoripares eccrines ne possède pas en soit d'odeur, elle peut, dans certaines conditions de macération, être à l'origine d'infections bactériennes ou d'irritations.

Les glandes sudoripares apocrines se situent notamment sous les aisselles. Elles sont plus grosses que les glandes eccrines et leur conduit excréteur débouche dans un follicule pileux.

Outre les composants de base identiques à ceux de la sueur excréter par les glandes eccrines, la sueur excrétée par les glandes apocrines contient également des molécules organiques (lipides et protéines) dont des phéromones qui, une fois transformées par des bactéries cutanées, sont à l'origine des odeurs dites « de transpiration ».

Pour prévenir l'apparition de ces mauvaises odeurs, on utilise notamment des compositions déodorantes plus classiquement désignées par « déodorants ». Les déodorants peuvent agir de diverses manières :
- en masquant les mauvaises odeurs, par exemple grâce à des ingrédients parfumés ;
- en absorbant la sueur et en limitant la diffusion des molécules « odorantes », par exemple grâce à du talc ;
- en agissant directement sur les bactéries qui, en métabolisant des constituants de la sueur apocrine, dégagent l'odeur attribuée à la transpiration.

Pour la préparation de compositions déodorantes efficaces, il est donc utile d'identifier des composés agissant directement sur les bactéries responsables des odeurs dites de « transpiration ».

La demande de brevet internationale WO 91/07165 décrit l'utilisation de dérivés benzyl benzoates de formule générale 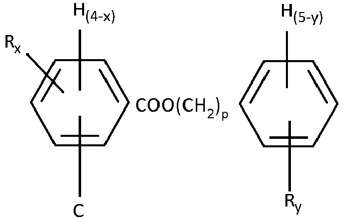 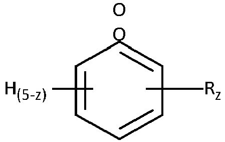 comme actifs antibactériens permettant de lutter contre les mauvaises odeurs.
Cette demande de brevet décrit en particulier le benzyl 4-benzoyloxybenzoate et son utilisation pour la préparation de compositions déodorantes.

Dans Synthesis and Properties of Flexible Poly(ether ketone) Backbones, Grafted with Stiff Monodisperse Sides Chaines, Macromolecules, 1998, 31, 6559-6565, les auteurs décrivent une voie de synthèse permettant de préparer le benzyl-4-(benzoyloxy-1,4-benzoyloxy)benzoate. Néanmoins, aucune propriété particulière n'est attribuée à ce composé.

Ces dernières années, outre l'efficacité « intrinsèque » des compositions déodorantes, l'accent a également été mis sur leur durabilité, c'est-à-dire sur la durée durant laquelle l'actif déodorant est susceptible de prévenir efficacement l'apparition de mauvaises odeurs.

Dans le cadre du développement de nouvelles compositions déodorantes il est donc utile d'identifier des composés permettant de lutter efficacement contre l'apparition des mauvaises odeurs pendant une durée prolongée en comparaison des actifs déjà existants.

Or, il a maintenant été trouvé de nouveaux dérivés benzyl benzoates qui, de façon tout à fait surprenante, possèdent non seulement une efficacité « intrinsèque » équivalente à celles des actifs déodorants existants, mais également une durée d'action améliorée en comparaison de ces mêmes actifs.

La présente invention a donc pour objet un composé de formule générale (I) 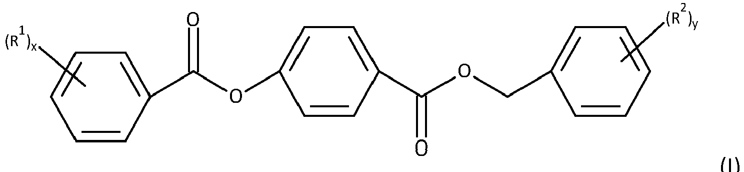 dans laquelle :
- x est un entier égal à 1, 2, 3, 4 ou 5 ;
- y est un entier égal à 0, 1, 2, 3, 4 ou 5 ;
- chaque substituant R¹ est choisi indépendamment des autres comme étant un groupe phenoxy, éventuellement substitué par un ou plusieurs substituants choisis indépendamment les uns des autres comme étant un groupe hydroxy, C₁-C₆-alkyle, C₁-C₆-hydroxyalkyle, C₁-C₆-alkoxy ou C₁-C₆-alkylcarbonyloxy ; et
- chaque substituant R² est choisi indépendamment des autres comme étant un groupe phenoxy, phenylC₁-C₆-alkoxy ou phenylC₁-C₆-alkylcarbonyloxy, chacun de ces groupes pouvant éventuellement être substitué par un ou plusieurs substituants choisis indépendamment les uns des autres comme étant un groupe hydroxy, C₁-C₆-alkyle, C₁-C₆-hydroxyalkyle, C₁-C₆-alkoxy ou C₁-C₆-alkylcarbonyloxy ;
à l'exception du benzyl-4-(benzoyloxy-1,4-benzoyloxy)benzoate.

Les composés selon la présente invention possèdent des propriétés déodorantes comparables à celles des actifs existants et présentent de plus une efficacité durable et prolongée.

Dans le cadre de la présente invention :
- on entend par « C₁-C₆-alkyle » une chaîne hydrocarbonée saturée, linéaire ou ramifiée, et comportant de 1 à 6 atomes de carbone, notamment le groupe méthyle, ou éthyle ;
- on entend par « C₁-C₆-alkoxy » un groupe -O-(Cₓ-C_{y}-alkyle), notamment le groupe methoxy ou ethoxy ;
- on entend par « phenylC₁-C_{y}-alkoxy » un groupe -O-(Cₓ-C_{y}-alkyle)Ph, notamment le groupe phenylmethoxy ou phenylethoxy;
- on entend par « phenylCₓ-C_{y}-alkylcarbonyloxy » un groupe -O-(C=O)-(Cₓ-C_{y}-alkyle)Ph, notamment le groupe benzoyloxy;
- le terme « alkyle » tel que défini ci-dessus conserve la même définition quand il intègre le nom d'un groupe tel que par exemple hydroxyalkyle ou alkylcarbonyloxy ;
- on entend par « déodorant » tout produit ou composition cosmétique destiné à prévenir, masquer, éliminer ou réduire les mauvaises odeurs corporelles en agissant notamment sur les bactéries qui, en métabolisant des constituants de la sueur apocrine, dégagent l'odeur qu'on attribue à la transpiration ;
- on entend par « traitement déodorant » tout traitement cosmétique comprenant l'application d'un ou plusieurs principes actifs déodorants en vue de prévenir masquer, éliminer ou réduire l'apparition des mauvaises odeurs corporelles.

De préférence, la présente invention a pour objet un composé de formule générale (I) tel que défini précédemment dans lequel les caractéristiques suivantes sont choisies seules ou en combinaison :
- x est un entier égal à 1, 2 ou 3 ; de préférence encore, x est égal à 1 ou 2 ; de façon tout à fait préférée, x est égal à 1 ;
- y est un entier égal à 0, 1 ou 2 ; de préférence encore, y est égal à 0 ou 1; de façon tout à fait préférée, y est égal à 0 ;
- chaque substituant R¹ est choisi indépendamment des autres comme étant un groupe phenoxy éventuellement substitué par un ou plusieurs substituants choisis indépendamment les uns des autres comme étant un groupe hydroxy, C₁-C₆-alkyle, C₁-C₆-hydroxyalkyle, C₁-C₆-alkoxy ou C₁-C₆-alkylcarbonyloxy; de préférence encore, chaque substituant R¹ est choisi indépendamment des autres comme étant un groupe phenoxy ou benzoyloxy, chacun de ces groupes pouvant éventuellement être substitué par un ou plusieurs groupes hydroxy; de façon tout à fait préférée, chaque substituant R¹ est choisi indépendamment des autres comme étant un groupe phénoxy éventuellement substitué par un ou plusieurs groupes hydroxy ;
- chaque substituant R² est choisi indépendamment des autres comme étant un groupe phenoxy ou benzoyloxy, chacun de ces groupes pouvant éventuellement être substitué par un ou plusieurs substituants choisis indépendamment les uns des autres comme étant un groupe hydroxy, C₁-C₆-alkyle, C₁-C₆-hydroxyalkyle, C₁-C₆-alkoxy ou C₁-C₆-alkylcarbonyloxy; de préférence encore, chaque substituant R² est choisi indépendamment des autres comme étant un groupe phenoxy ou benzoyloxy, chacun de ces groupes pouvant éventuellement être substitué par un ou plusieurs groupes hydroxy; de façon tout à fait préférée, chaque substituant R² est choisi indépendamment des autres comme étant un groupe phénoxy éventuellement substitué par un ou plusieurs groupes hydroxy.

De façon préférée, la présente invention a pour objet un composé de formule générale (I-a) 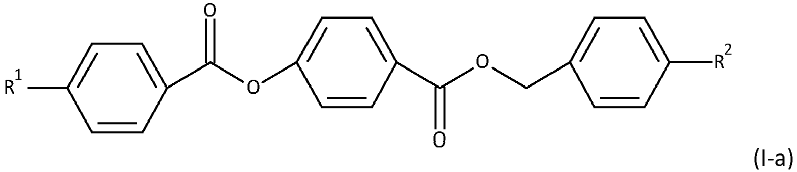 dans laquelle R¹ et R² sont tels que définis précédemment.

De façon préférée, la présente invention a également pour objet un composé de formule générale (I-b) 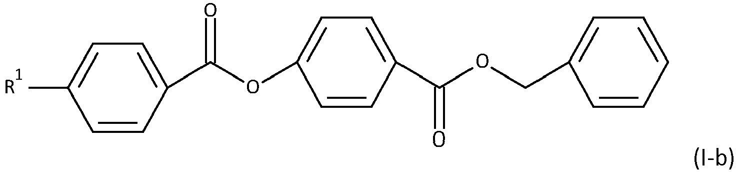 dans laquelle R¹ est tel que défini précédemment.

Les composés de formule (I) peuvent être préparés par tout procédé connu et classiquement utilisé par l'homme du métier.

A titre d'exemple, les composés de formule (I) selon la présente invention peuvent être préparés selon le schéma réactionnel suivant :

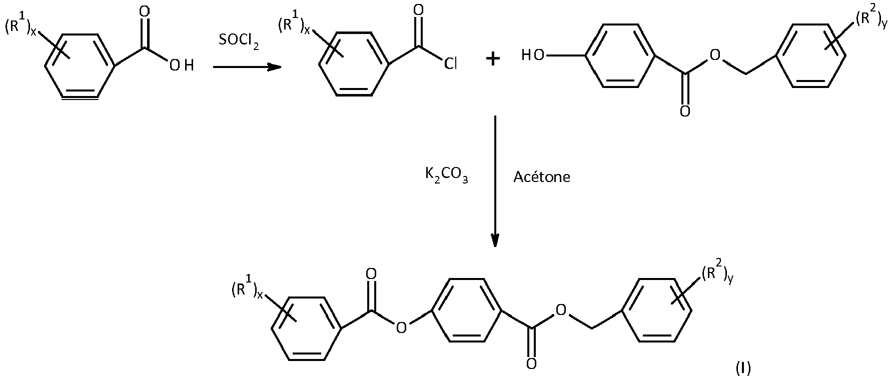

Les composés de formule (I) selon la présente invention peuvent donc être utilisés en cosmétique pour le traitement déodorant.

La présente invention a donc également pour objet l'utilisation cosmétique d'un ou plusieurs composés de formule (I) tels que définis précédemment comme déodorant.

La présente invention a également pour objet une composition cosmétique comprenant (à titre de principe actif) un ou plusieurs composés de formule (I) tels que définis précédemment, ainsi que son utilisation comme déodorant.

Les compositions cosmétiques selon la présente invention peuvent être formulées sous toute forme galénique appropriée à leur administration. Les compositions selon la présente invention peuvent ainsi être formulées sous forme de poudres, crème, gel, lotion, lait, émulsion huile dans eau ou eau dans huile, parfum, solution, onguent, pulvérisateur, huile corporelle, lotion après-rasage, savon ou bâton.

Les compositions cosmétiques selon la présente invention contiennent un ou plusieurs composés de formule (I) selon la présente invention dans des teneurs allant de 0,005% à 25% en poids total de la composition, préférentiellement de 0,01% à 15% en poids total de la composition, préférentiellement encore de 0,05% à 5% en poids total de la composition.

Pour la préparation de ces compositions cosmétiques, un ou plusieurs composés de formule (I) selon la présente invention sont mélangés aux excipients classiquement utilisés dans le domaine cosmétique.

Les compositions cosmétiques selon la présente invention peuvent prendre la forme d'un parfum comprenant un ou plusieurs composés de formule (I) selon la présente invention. Le parfum ainsi préparé peut être lui-même incorporé dans une autre composition cosmétique.

Les compositions cosmétiques selon la présente invention peuvent prendre la forme d'une crème dans laquelle un ou plusieurs composés de formule (I) selon la présente invention sont associés aux excipients couramment utilisés en cosmétologie.

Les compositions cosmétiques selon la présente invention peuvent prendre la forme de gels dans les excipients appropriés tels que les esters de cellulose ou d'autres agents gélifiants, tels que Carbopol, Sepinov (polyacrylate), la gomme guar.

Les compositions cosmétiques selon la présente invention peuvent aussi prendre la forme d'une lotion ou d'une solution dans lesquelles un ou plusieurs composés de formule (I) selon la présente invention sont sous forme encapsulée.

Les microsphères suivant l'invention peuvent par exemple être constituées de corps gras, d'agar et d'eau. Un ou plusieurs composés de formule (I) selon la présente invention peuvent être incorporés dans des vecteurs de type liposomes, glycosphères, cyclodextrines, dans des chylomicrons, des macro-, micro-, nano-particules ainsi que les macro-, micro- et nanocapsules et aussi être absorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50°C sans qu'il y ait sédimentation des constituants ou séparation des phases.
Les compositions cosmétiques selon la présente invention peuvent aussi contenir des additifs ou des adjuvants usuels en cosmétologie, comme par exemple des agents antimicrobiens ou des parfums mais aussi des lipides d'extraction ou de synthèse, des polymères gélifiants et viscosants, des tensio-actifs et des émulsifiants, des principes actifs hydro- ou liposolubles, des extraits de plantes, des extraits tissulaires, des extraits marins, des actifs de synthèse.

Les compositions cosmétiques selon la présente invention peuvent aussi comprendre d'autres principes actifs complémentaires choisis pour leur action. Lorsque les compositions selon la présente invention contiennent des principes actifs complémentaires, ceux-ci sont généralement présents dans la composition à une concentration suffisamment élevée pour qu'ils puissent exercer leur activité.

Les compositions cosmétiques selon la présente invention sont de préférence utilisées quotidiennement et appliquées une ou plusieurs fois par jour.

La présente invention est illustrée de manière non limitative par les exemples suivants.

### Exemple 1 : Composés selon l'invention

| **Composé** | **P.f. (°C)** |
|---|---|
| 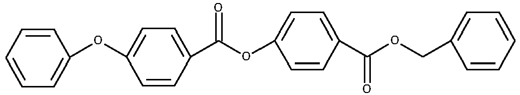 | 112-120 |
| *Composé 1* | |

### Exemple 2 : Préparation du benzyl[4-(4-phenoxy)benzoyloxy]benzoate (composé 1)

### 2.1- Préparation du chlorure de 4-phenoxybenzoyle

10,7 g (50 mmol) d'acide 4-phénoxybenzoïque (CAS # 2215-77-2; Aldrich 246182) sont mis en suspension dans 75 ml de chlorure de thionyle (CAS # 7719-09-7).

Sous agitation magnétique, on chauffe progressivement. La solubilisation totale est obtenue lorsque la température de 60 °C est atteinte. L'ensemble est maintenu sous un léger reflux pendant 45 minutes, puis, on évapore à sec au Rotavapor.

L'huile jaune obtenue est le chlorure de 4-phenoxybenzoyle.

### 2.2 - Préparation du benzyl[4-(4-phenoxy)benzoyloxylbenzoate

Dans un ballon de 250 ml on place 11,4 g (50 mmol) de 4-hydroxybenzoate de benzyle (CAS # 94-18-8; Aldrich 300709), 55 ml d'acétone (CAS # 67-64-1) et 6,9 g (50 mmol) de carbonate de potassium anhydre (CAS # 584-08-7).

L'ensemble est mis sous agitation magnétique et on ajoute rapidement la totalité du chlorure de 4-phenoxybenzoyle obtenu à l'étape 2.1 préalablement mis en solution dans 100 ml d'acétone.

Au bout de 2h15, l'agitation est rendue impossible par la formation d'un bloc de couleur blanche.

Le bloc est finement divisé puis dilué par 100 ml supplémentaires d'acétone. Le mélange est versé dans un excès d'eau. La solution est acidifiée par de l'acide chloridrique concentré (CAS # 7647-01-0) et extraite par 2 x 200 ml de dichlorométhane.

Les phases organiques réunies sont évaporées à sec pour donner 26 g d'un solide blanc.

Une filtration sur 30 parties de silice (Kieselgel 60A, 70-230 mesh, Fluka) suivie d'une élution au dichlorométhane puis réunion des fractions pures (1) et concentration jusqu'au début de cristallisation, dilution au pentane, essorage et lavage au pentane, fournit 15,85 g de cristaux blancs de (rendement 74,8%).

### Exemple 3 - Activité déodorante et durabilité

### Protocole

Le test est réalisé *in vitro.*

Le composé 1 et le benzyl-4-benzoyloxybenzoate (composé de référence décrit dans la demande de brevet internationale WO 91/07165) sont tous deux dilués à 10% dans le benzylbenzoate, et incorporés dans un milieu de culture spécifique au moment de la répartition en flacons de 50 ml.
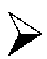 La composition des flacons se caractérise comme suit :Flacon n°1
   ∘ Brain Heart Infusion (médium) + 0.5% Tween 40
   ∘ Composé 1 ou benzyl-4-benzoyloxybenzoate - 0.25%
   ∘ Enzyme (Lipozyme® CALB, Novozymes) - 2%
   ∘ Bactéries *(Corynaebacterium xerosis)* - environ 106 UFC/ml
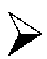 Flacon n°2
   ∘ Brain Heart Infusion (médium) + 0.5% Tween 40
   ∘ Composé 1 ou benzyl-4-benzoyloxybenzoate - 0.25%
   ∘ Bactéries (*Corynaebacterium xerosis*) - - environ 106 UFC/ml
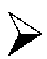 Flacon n°3
   ∘ Brain Heart Infusion (médium) + 0.5% Tween 40
   ∘ Composé 1 ou benzyl-4-benzoyloxybenzoate - 0.25%
   ∘ Enzyme (Lipozyme® CALB, Novozymes) - 2%

Les flacons sont ensuite incubés sous agitation à 37°C pendant 48 heures.

Après 24 heures, un prélèvement est effectué, et 100µl d'une dilution au 1/1000ème sont étalés sur gélose. Un comptage du nombre d'UFC (Unité Formant Colonie) est effectué.

### Résultats

C'est la comparaison des ratios entre la quantité d'UFC/ml à un temps t pour le produit + enzyme et le produit seul qui permet l'évaluation de l'activité inhibitrice du produit.

Les résultats obtenus sont rapportés dans le Tableau ci-dessous.

| **N° du flacon / Composé** | **Nombre d'UFC/ml à t +24h** | **Nombre d'UFC/ml à t+48h** |
|---|---|---|
| Flacon 1 / Composé 1 | 10⁷ | 36000 |
| Flacon 1 / benzyl-4-benzoyloxybenzoate | 2.7x10⁶ | 10⁸ |
| Flacon 2 / Composé 1 | 10⁸ | 3.2x10⁶ |
| Flacon 2 / benzyl-4-benzoyloxybenzoate | 10⁸ | 10⁹ |
| Flacon 3 / Composé 1 | 0 | 0 |
| Flacon 3 / benzyl-4-benzoyloxybenzoate | 0 | 0 |

Les résultats obtenus mettent clairement en évidence :
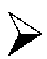 une activité déodorante comparable du composé et du benzyl-4-benzoyloxybenzoate après 24 heures,
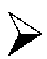 une activité déodorante significativement supérieure du composé 1 par rapport au benzyl-4-benzoyloxybenzoate après 48 heures.

## Revendications

1. Composé de formule générale (I) 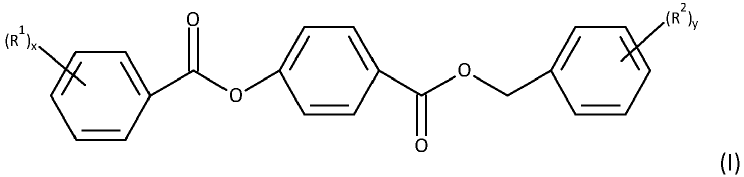 dans laquelle :
- x est un entier égal à 1, 2, 3, 4 ou 5 ;
- y est un entier égal à 0, 1, 2, 3, 4 ou 5 ;
- chaque substituant R¹ est choisi indépendamment des autres comme étant un groupe phenoxy éventuellement substitué par un ou plusieurs substituants choisis indépendamment les uns des autres comme étant un groupe hydroxy, C₁-C₆-alkyle, C₁-C₆-hydroxyalkyle, C₁-C₆-alkoxy ou C₁-C₆-alkylcarbonyloxy ; et
- chaque substituant R² est choisi indépendamment des autres comme étant un groupe phenoxy, benzoyloxy, phenylC₁-C₆-alkoxy ou phenylC₁-C₆-alkylcarbonyloxy, chacun de ces groupes pouvant éventuellement être substitué par un ou plusieurs substituants choisis indépendamment les uns des autres comme étant un groupe hydroxy, C₁-C₆-alkyle, C₁-C₆-hydroxyalkyle, C₁-C₆-alkoxy ou C₁-C₆-alkylcarbonyloxy.

2. Composé selon la revendication 1 **caractérisé en ce que** x est égal à 1, 2 ou 3.

3. Composé selon la revendication 1 ou 2, **caractérisé en ce que** y est égal à 0, 1 ou 2.

4. Composé selon selon l'une quelconque des revendications 1 à 3, **caractérisé en ce que** chaque substituant R¹ est choisi indépendamment des autres comme étant un groupe phenoxy éventuellement substitué par un ou plusieurs groupes hydroxy.

5. Composé selon l'une quelconque des revendications 1 à 4, **caractérisé en ce que** chaque substituant R² est choisi indépendamment des autres comme étant un groupe phenoxy ou benzoyloxy, chacun de ces groupes pouvant éventuellement être substitué par un ou plusieurs substituants choisis indépendamment les uns des autres comme étant un groupe hydroxy, C₁-C₆-alkyle, C₁-C₆-hydroxyalkyle, C₁-C₆-alkoxy ou C₁-C₆-alkylcarbonyloxy.

6. Composé de formule générale (I-a) 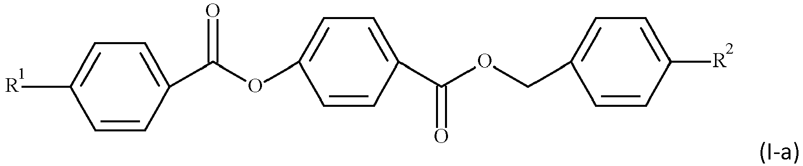 dans laquelle R¹ et R² sont tels que définis dans l'une des revendications précédentes.

7. Composé de formule générale (I-b) 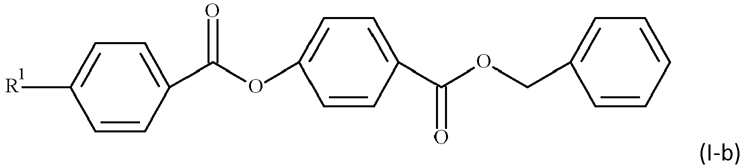 dans laquelle R¹ est tel que défini dans l'une des revendications précédentes.

8. Procédé de préparation d'un composé selon l'une des revendications 1 à 7 selon le schéma réactionnel suivant :

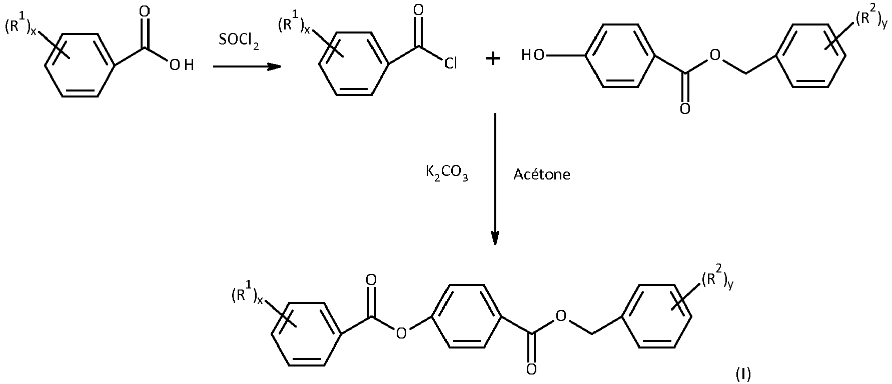

9. Composition cosmétique comprenant un composé selon l'une quelconque des revendications 1 à 7.

10. Utilisation d'une composition selon la revendication 9 comme déodorant.

## Patentansprüche

1. Verbindung mit der allgemeinen Formel (I) 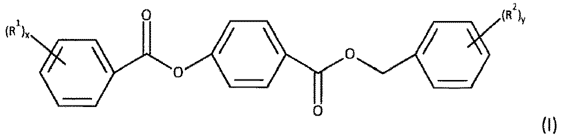 wobei:
- x eine Ganzzahl gleich 1, 2, 3, 4 oder 5 ist;
- y eine Ganzzahl gleich 0, 1, 2, 3, 4 oder 5 ist;
- jeder Substituent R¹ unabhängig von den anderen als Phenoxygruppe ausgewählt ist, gegebenenfalls substituiert durch einen oder mehrere Substituenten, unabhängig voneinander ausgewählt als Hydroxy-, C₁-C₆-Alkyl-, C₁-C₆-Hydroxyalkyl-, C₁-C₆-Alkoxy- oder C₁-C₆-Alkylcarbonyloxygruppe; und
- jeder Substituent R² unabhängig von den anderen als Phenoxy-, Benzyloxy-, PhenylC₁-C₆-Alkoxy oder PhenylC₁-C₆-Alkylcarbonyloxygruppe ausgewählt ist, wobei jede dieser Gruppen gegebenenfalls durch einen oder mehrere Substituenten substituiert werden kann, unabhängig voneinander ausgewählt als Hydroxy-, C₁-C₆-Alkyl-, C₁-C₆-Hydroxyalkyl-, C₁-C₆-Alkoxy- oder C₁-C₆-Alkylcarbonyloxygruppe.

2. Verbindung nach Anspruch 1, **dadurch gekennzeichnet, dass** x gleich 1, 2 oder 3 ist.

3. Verbindung nach Anspruch 1 oder 2, **dadurch gekennzeichnet, dass** y gleich 0, 1 oder 2 ist.

4. Verbindung nach einem der Ansprüche 1 bis 3, **dadurch gekennzeichnet, dass** jeder Substituent R¹ unabhängig von den anderen als Phenoxygruppe ausgewählt ist, gegebenenfalls substituiert durch eine oder mehre Hydroxygruppen.

5. Verbindung nach einem der Ansprüche 1 bis 4, **dadurch gekennzeichnet, dass** jeder Substituent R² unabhängig von den anderen als Phenoxy- oder Benzoyloxygruppe ausgewählt ist, wobei jede dieser Gruppen gegebenenfalls durch einen oder mehrere Substituenten substituiert werden kann, unabhängig voneinander ausgewählt als Hydroxy-, C₁-C₆-Alkyl-, C₁-C₆-Hydroxyalkyl-, C₁-C₆-Alkoxy- oder C₁-C₆-Alkylcarbonyloxygruppe.

6. Verbindung mit der allgemeinen Formel (I-a) 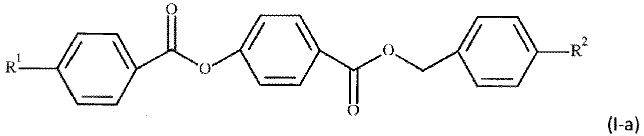 wobei R¹ und R² solcherart sind, wie in einem der vorhergehenden Ansprüche definiert.

7. Verbindung mit der allgemeinen Formel (I-b) 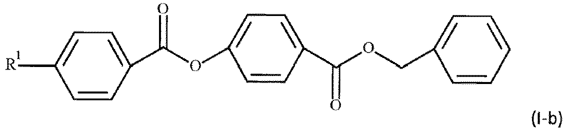 wobei R¹ solcherart ist, wie in einem der vorhergehenden Ansprüche definiert.

8. Verfahren zur Herstellung einer Verbindung nach einem der Ansprüche 1 bis 7 gemäß dem folgenden Reaktionsschema:

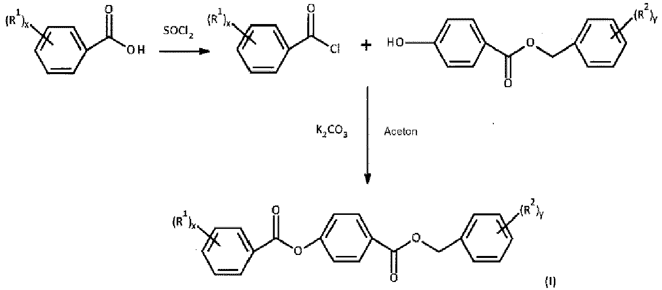

9. Kosmetische Zusammensetzung, umfassend eine Verbindung nach einem der Ansprüche 1 bis 7.

10. Verwendung einer Zusammensetzung nach Anspruch 9 als Deodorant.

## Claims

1. Compound of general formula (I) 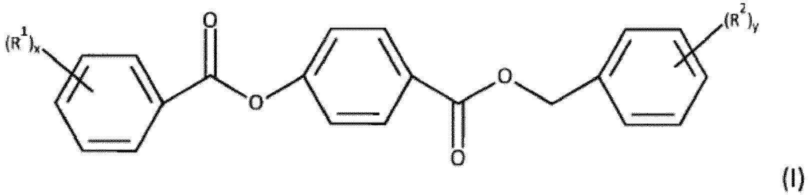 wherein:
- x is an integer equal to 1, 2, 3, 4 or 5;
- y is an integer equal to 0, 1, 2, 3, 4 or 5;
- each substituent R¹ is chosen independently from the others as being a phenoxy group optionally substituted with one or more substituent(s) selected independently from each other as a hydroxy, C₁-C₆-alkyl, C₁-C₆-hydroxyalkyl, C₁-C₆-alkoxy or C₁-C₆-alkylcarbonyloxy group; and
- each substituent R² is chosen independently from the others as being a phenoxy, benzoyloxy, phenylC₁-C₆-alkoxy or phenylC₁-C₆-alkylcarbonyloxy group, each of these groups may be optionally substituted with one or several substituent(s) selected independently from each other as a hydroxy, C₁-C₆-alkyl, C₁-C₆-hydroxyalkyl, C₁-C₆-alkoxy or C₁-C₆-alkylcarbonyloxy group.

2. Compound according to claim 1 **characterized in that** x is equal to 1, 2 or 3.

3. Compound according to claim 1 or 2, **characterized in that** y is equal to 0, 1 or 2.

4. Compound according to any one of claims 1 to 3, **characterized in that** each substituent R¹ is chosen independently from the others as being a phenoxy group optionally substituted with one or several hydroxy group(s).

5. Compound according to any one of claims 1 to 4, **characterized in that** each substituent R² is chosen independently from the others as being a phenoxy, benzoyloxy group, each of these groups may be optionally substituted with one or several substituent(s) selected independently from each other as a hydroxy, C₁-C₆-alkyl, C₁-C₆-hydroxyalkyl, C₁-C₆-alkoxy or C₁-C₆-alkylcarbonyloxy group.

6. Compound of general formula (I-a) 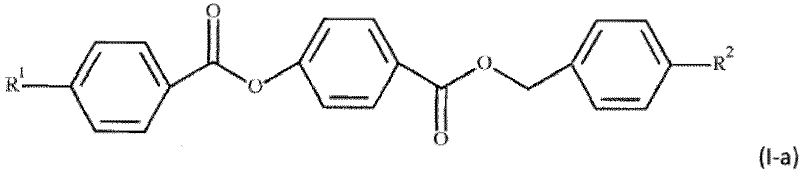 wherein R¹ and R² are as defined in any of the preceding claims.

7. Compound of general formula (I-b) 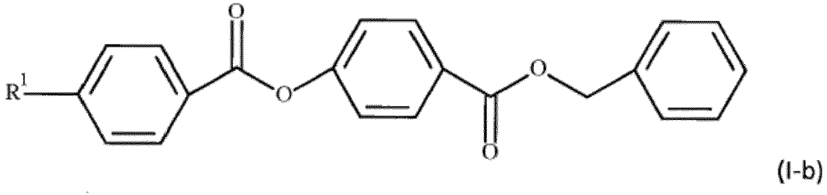 wherein R¹ is as defined in any of the preceding claims.

8. Process for preparing a compound according to any of claims 1 to 7 according to the following reaction scheme:

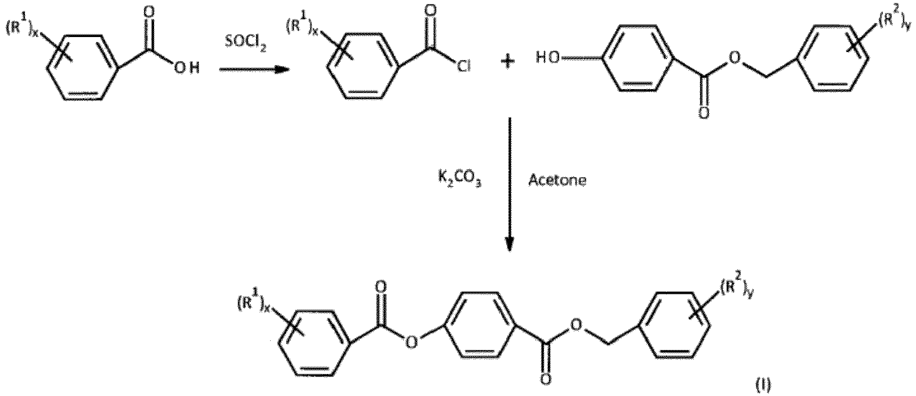

9. Cosmetic composition comprising a compound according to any one of claims 1 to 7.

10. Use of a composition according to claim 9 as a deodorant.
